# EUROPEAN PATENT SPECIFICATION

(11) **EP 3 393 361 B1**
(45) Date of publication and mention of the grant of the patent: **17.08.2022**
(21) Application number: 16816281.6
(22) Date of filing: 20.12.2016
(51) Int. Cl.: A61B 6/08, A61B 6/00, A61B 6/03

(54) **CT PERFUSION PROTOCOL TARGETING**
ABZIELUNG AUF CT-PERFUSIONSPROTOKOLL
CIBLAGE DE PROTOCOLE DE PERFUSION PAR TOMOGRAPHIE ASSISTÉE PAR ORDINATEUR

(30) Priority: 22.12.2015 US 201562270847 P
(43) Date of publication of application: 31.10.2018
(73) Proprietor: Koninklijke Philips N.V., 5656 AG Eindhoven (NL)
(72) Inventor: PREVRHAL, Sven, 5656 AE Eindhoven (NL); SCHMITT, Holger, 5656 AE Eindhoven (NL); NICKISCH, Hannes, 5656 AE Eindhoven (NL)
(74) Representative: Philips Intellectual Property & Standards
(86) International application number: PCT/EP2016/081904
(87) International publication number: WO 2017/108779

(56) References cited:
- WO-A2-2009/109905
- US-A1- 2010 272 344
- US-A1- 2015 250 395
- BELIVEAU P ET AL: "Computation of coronary perfusion territories from CT angiography", COMPUTERS IN CARDIOLOGY, 2007, IEEE, PISCATAWAY, NJ, USA, 30 September 2007 (2007-09-30), pages 753-756, XP031404821, ISBN: 978-1-4244-2533-4
- AKOS VARGA-SZEMES ET AL: "CT Myocardial Perfusion Imaging", AMERICAN JOURNAL OF ROENTGENOLOGY, vol. 204, no. 3, 1 March 2015 (2015-03-01) , pages 487-497, XP055357167, US ISSN: 0361-803X, DOI: 10.2214/AJR.14.13546
- Franziska Dorn ET AL: "Order of CT stroke protocol (CTA before or after CTP): impact on image quality", NEURORADIOLOGY, vol. 54, no. 2, 23 February 2011 (2011-02-23), pages 105-112, XP055643661, DE ISSN: 0028-3940, DOI: 10.1007/s00234-011-0840-8

## Description

### FIELD OF THE INVENTION

The following generally relates to medical imaging with specific application to computed tomography (CT) perfusion scanning, such as perfusion scanning of organs, such as a heart, a liver or a brain.

### BACKGROUND OF THE INVENTION

A CT perfusion protocol uses a CT scanning device to scan the myocardium of a heart during uptake of an administered contrast agent to identify and diagnose myocardial defects. During the perfusion scan a CT rotating gantry scans at a same axial position or z-axis with a portion of the heart in the field of view to observe the rise of contrast in the tissues. An X-ray source emits x-rays that traverse the tissues and are detected by a radiation detector. The detected x-rays are reconstructed into CT images that are then fused to a perfusion image. The same axial position corresponds to a position along a longitudinal axis of the patient, e.g. patent remains in the same position relative to the plane of the rotating gantry. The rise of contrast in the myocardial tissues occurs in seconds. Missing the uptake period can mean waiting for washout and re-administering the contrast agent, and exposing a patient to additional ionizing radiation from the CT scanning device. The CT scanning device is typically configured to monitor for the contrast agent with a lower dose scan before switching to a higher dose with higher resolution during the uptake.

Typical CT scanning devices have a field of view sufficient only to scan a portion of the heart. One approach to ensuring the scanning potentially damaged myocardium is to manufacture the CT scanning device with a large field of view or scan coverage sufficient to repeatedly scan the entire heart at the same axial position. With the entire heart being scanned in each rotation, the uptake in all of the myocardium can be observed. However, this also means that a larger portion of tissues including the entire heart are subjected to a larger dose of ionizing radiation during monitoring and scanning, and ionizing radiation is an accumulated dose for the patient. Another approach is to use a shuttle mode where a patient in moved in a limited field of view at incremental positions, e.g. back and forth between two positions, to expand the scan coverage. However, the shuttle mode increases the dose and can miss the peak enhancement during uptake.

Myocardial defects, such as damaged heart muscle, are typically caused by lack of oxygen to the myocardial tissues attributable to blockages in the arterial supply to the myocardial tissues, e.g. atherosclerotic coronary artery disease (CAD).

US2010/272344 A1 discloses a CT-data acquiring unit that acquires region-of-interest information that is set as the ischemic region on the CT image by the operator. When an ischemic region is detected in a myocardium through a diagnosis by an X-ray CT apparatus, the detected ischemic region is to be displayed in a highlighted manner on an X-ray myocardium perfusion image.

Beilveau et.al. disclose, in "Computation of coronary perfusion territories for CT angiography, Computers in Cardiology', 2007, IEEE, 30 September 2007, pages 753-756, a technique to automatically generated patient specific coronary distribution maps based on noninvasive multi-slice computed tomography (MSCT).

US2015250395 A1 discloses the preamble of claim 1 and the corresponding method steps.

### SUMMARY OF THE DISCLOSURE

The invention is defined in the claims.

Aspects described herein address the above-referenced problems and others.

The following describes a method and system for targeting the CT perfusion scan coverage of a portion of an organ, such as the heart, the liver or the brain. The portion of the organ imaged during the perfusion scan is identified according to spatially located stenosis and a feeding territory map of the organ tissues. In one embodiment, the stenosis can include a stent location. In one embodiment, the stenosis is spatially located using a CT angiography scan.

In one aspect, a system for a targeted perfusion scan includes a computed tomography (CT) scanner, a feeding territory map and a targeted perfusion unit. The CT scanner performs a perfusion scan of a portion of tissues of an organ. The feeding territory map maps arterial locations of an arterial vessel tree to spatially located organ tissues of the organ fed by the arterial locations. The targeted perfusion unit includes one or more processors configured to determine targeted coverage from a location of a stenosis and the feeding territory map, and to control the CT scanner to perform the perfusion scan according to the determined targeted coverage.

In another aspect, a method of targeting a perfusion scan includes determining targeted coverage from a location of a stenosis and a feeding territory map. A computed tomography (CT) scanner is controlled to scan a portion of organ tissues according to the targeted coverage during the perfusion scan.

In another aspect, a non-transitory computer readable storage medium contains instructions that when executed by one or more processors determine a targeted coverage of a computed tomography (CT) scanner from a location of a stenosis in an arterial vessel tree of an organ and a feeding territory map that maps arterial locations of the arterial vessel tree to spatially located organ tissues of the organ fed by the arterial locations. The instructions further control the CT scanner to perform a targeted CT perfusion (CTP) scan according to the targeted coverage.

### BRIEF DESCRIPTION OF THE DRAWINGS

The drawings are only for purposes of illustrating the preferred embodiments and are not to be construed as limiting the invention.
FIGURE 1 schematically illustrates an embodiment of a targeted perfusion protocol system.
FIGURE 2 illustrates an exemplary heart scan targeted coverage with a stenosis and corresponding perfusion defect.
FIGURE 3 flowcharts an embodiment of a method of targeting perfusion scanning coverage.

### DETAILED DESCRIPTION OF EMBODIMENTS

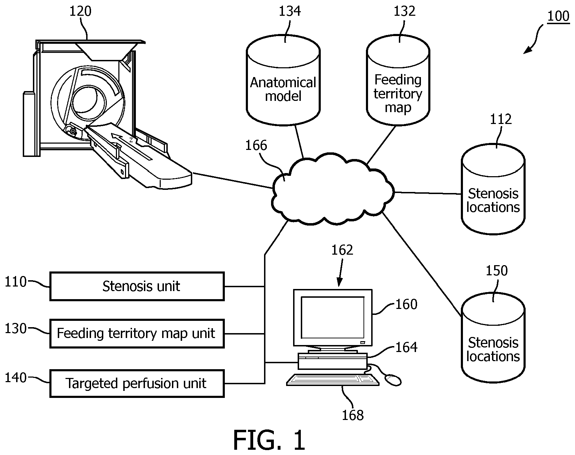

Initially referring to FIGURE 1, a targeted perfusion protocol system 100 is schematically illustrated. A stenosis unit 110 identifies stenosis locations 112 in an organ of a patient, such as a heart. The stenosis is an abnormal narrowing in the arterial lumen, such as in the coronary artery of the heart. In one embodiment, the stenosis is identified from a prior volumetric image of the organ, e.g. prior imaging study. In one embodiment, the stenosis can include a stent location. In one embodiment, the stenosis unit 110 controls a scanning device 120, such as a CT scanner, an x-ray scanner, a magnetic resonance (MR) scanner, combinations and the like, to scan the heart according to an angiography protocol. The scanning device 120 according to the angiography protocol generates the location(s) of stenosis 112, such as in a volumetric image of the organ, using techniques known in the art. For example, a CT angiography (CTA) protocol scans the entire heart in a helical CT scan, which is a lower dose scan than a perfusion scan. The scan can include a contrast agent, which contrasts the arterial vessel. A coronary artery vessel tree is segmented. Diameters along the vessel tree can be measured and a fractional flow reserve (FFR) computed (FFR-CT). Based on the spatially located segmented arterial vessel, diameters, and FFR-CT, a location of stenosis is identified.

A feeding territory map unit 130 maps the location of the stenosis 112 to positions of a CT perfusion scan coverage using a feeding territory map 132. A feeding territory map associates locations or points along an arterial vessel tree with spatial locations of the organ tissue. The associated locations along the arterial vessel tree correspond to tissues fed by portions of arterial vessel tree downstream of arterial blood flow. For example, a branch along the coronary artery vessel tree is mapped to myocardial tissue supplied with arterial blood flow by the branch. The feeding territory map 132 can be initially constructed from a sampling of one or more healthy patient hearts scanned with a perfusion protocol, and associating branches with specific areas of myocardial tissue. In one embodiment, the feeding territory can be localized to a 2-3 centimeter (cm) axial or z coverage, or smaller depending on the size of the arterial branch. In one embodiment, the initially constructed map can be further refined or fit to a prior imaged organ of the patient using an anatomical model 134. The anatomical model 134 can deformably dimension the feeding territory map 132 using techniques known in the art, such as triangular mesh surfaces.

The feeding territory map unit 130 can map the locations of the stenosis 112 identified in the prior or generated image, e.g. a CTA image, using the dimensioned feeding territory map 132 or anatomically corresponding positions using a non-dimensioned feeding territory map 132 to portions of the organ tissues supplied downstream of the stenosis. In some instances, the CTA image is generated from a scan that uses one seventh or less ionizing radiation dose than a corresponding CT perfusion scan. The portions of the organ tissues supplied downstream of the stenosis are used to determine positional parameters of a targeted perfusion scan. For example, the feeding territory map 132 can include for each smallest branch of the arterial vessel tree, spatial locations of organ tissues, such as voxels corresponding to each smallest branch. The feeding territory map 132 can include an association of each smallest branch to larger segments and hierarchically to the whole arterial vessel tree. Thus, smallest branches in any branch with a stenosis can be identified hierarchically and in turn, the organ tissues, such as myocardium, identified.

With the dimensioned feeding territory map 132, the locations of the stenosis 112 correspond spatially with locations along the arterial vessel tree that associate with organ tissues. The organ tissues fed by downstream branches according to the dimensioned feeding territory map 132 also correspond spatially with tissues of the imaged organ of the patient used to spatially fit the dimensioned feeding territory map 132. The z positions of corresponding tissues of the patient are used as positional parameters for the targeted perfusion scan.

With the non-dimensioned feeding territory map 132, the locations of the stenosis 112 correspond to anatomical locations along the arterial vessel tree. The anatomical location of the stenosis along the arterial vessel tree, e.g. in the patient image, is identified that corresponds an anatomical location along the arterial vessel tree in the non-dimensioned feeding territory map 132. From the associated organ tissues fed in downstream branches, the anatomical locations of the associated organ tissues are determined and use to anatomically locate the corresponding organ tissues of the patient used as z positional parameters for the targeted perfusion scan.

A targeted perfusion unit 140 controls a CT scanner 120 to scan a portion of the patient organ using coverage determined from the feeding territory map 132. A scout and/or pilot scan can identify the organ location in the patient and positions of anatomical locations in the organ. The targeted perfusion unit 140 uses the positional information, such as anatomical positions in the scout scan along the z-axis of the scanner and corresponding positions determined from the feeding territory map 132 to position the patient in the field of view or coverage during uptake of a contrast agent during a perfusion scan. The feeding territory map 132 can provide a start position and an end position of the fed organ tissues. The field of view in a large coverage scanner, i.e. the coverage and hence, a dose, can be limited to the start and end positions. The field of view in a limited field of view scanner can be positioned to the start and end positions. The coverage can include a margin of error.

The targeted perfusion unit 140 generates a perfusion image of the scanned portion of the patient tissue. The scanned portion of the patient tissue is determined from the axial start and end positions of the fed tissues downstream of the stenosis based on the feeding territory map 132. The generated perfusion image can be stored in a storage subsystem 150, such as a Picture Archiving and Communication System (PACS), a Radiology Information System (RIS), Electronic Medical Record (EMR), and the like or displayed on a display device 160 of a computing device 162.

The stenosis unit 110, the feeding territory map unit 130, and the targeted perfusion unit 140 comprise one or more configured processors 164, e.g., a microprocessor, a central processing unit, a digital processor, and the like). The one or more configured processors 164 are configured to execute at least one computer readable instruction stored in a computer readable storage medium, which excludes transitory medium and includes physical memory and/or other non-transitory medium. The one or more processors 164 may also execute one or more computer readable instructions carried by a carrier wave, a signal or other transitory medium. The one or more processors 164 can include local memory and/or distributed memory. The one or more processors 164 can include hardware/software for wired and/or wireless communications over a network 166. For example, the lines indicate communications paths between the various components which can be wired or wireless. The one or more processors 164 can comprise the computing device 162, such as a desktop computer, a laptop computer, a body worn computing device, a smartphone, a tablet, and/or cooperative/distributed computing devices including one or more configured servers (not shown). The computing device 162 can include one or more input devices 168 which receive commands, such as identifying and/or confirming the locations of stenosis 112 in a volumetric image, displaying the perfusion scan generated image, displaying the feeding territory map 132, identifying coverage positions relative to the scout/pilot scan, confirming coverage, and the like.

The stenosis locations 112, the anatomical model 134, the feeding territory map 132 are represented as digital data sets stored on an electronic storage medium or computer memory. The digital data sets can include a Digital Imaging and Communications in Medicine (DICOM) format or other suitable image or volumetric format.

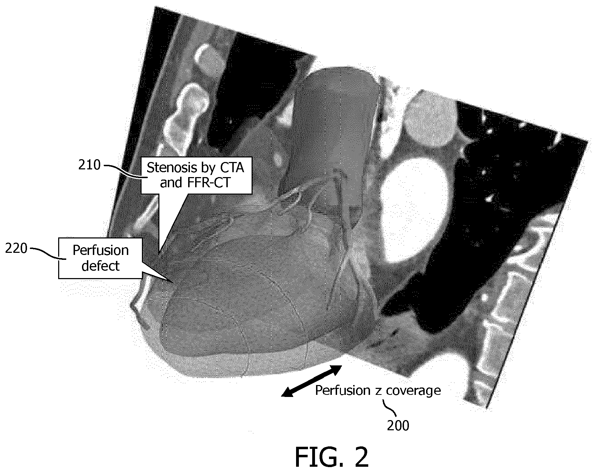

With reference to FIGURE 2, an exemplary heart scan targeted coverage 200 with a stenosis 210 and a corresponding perfusion defect 220 is illustrated in a cut-away two dimensional perspective view of the heart and the coronary artery. The stenosis 210 is identified from the CTA image and FFR-CT computation. The perfusion defect 220 is analyzed according the targeted perfusion scan. The location of the perfusion defect 220 is determined from the feeding territory map 132 based on the location of the stenosis 210, e.g. tissues fed by arterial branches downstream of the stenosis 210.

The targeted coverage 200, or axial start and end positions in the targeted perfusion scan, are determined from the corresponding axial limits of fed tissues downstream from the stenosis 210. For example, a start position corresponds to a first z position of the fed territory. The end position correspond to a second z position of the fed territory and the first and second z positions limit the coverage of the scan with the fed territory disposed in between the first and second z positions. In one embodiment, the first and/or the second z positions can include a margin of error, such as an additional distance adjacent to the fed territory. In some instances the margin of error can be measured based on the sampling of healthy hearts used to construct the feeding territory map 132.

In some instances, the targeted coverage 200 reduces the dose received by the patient by reducing the coverage of tissues in a perfusion scan, e.g. less than the entire organ. The targeted coverage 200 during a targeted perfusion scan includes the organ defect and the monitoring and scanning of the tissue defects during uptake of the contrast agent.

In some instances, more than one stenosis 210 is identified for a targeted perfusion scan. In one embodiment, the target coverage 200 is expanded to include corresponding axial positions for all fed tissues. In one embodiment, multiple targeted coverages are identified within the limits of the CT scanner 120 constraints. For example, a first targeted coverage of a first start position z^{s}₁ and a first end position z^{e}₁ corresponds to a first fed territory, and a second targeted coverage of a second start position z^{s}₂ and a second end position z^{e}₂ corresponds to a second fed territory. The first and second targeted coverage are within the field of view constraints of the CT scanner 120 and collimated in between. In another embodiment, the first and second targeted coverage are not within the field of view constraints of the CT scanner 120 and separate targeted perfusion scans are performed. In some instances, the locations of the fed tissues based on the feeding territory map 132 are used to prioritize the separate targeted perfusion scans.

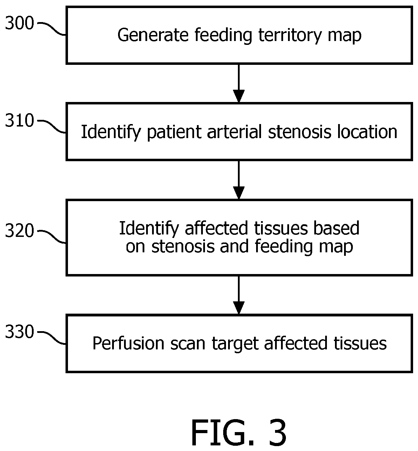

With reference to FIGURE 3, an embodiment of a method of targeting perfusion scanning coverage is flowcharted. At 300, a feeding territory map 132 is generated based on a sampling of one or more healthy organs, e.g. according to perfusion studies. The feeding territory map 132 includes the arterial vessel and the corresponding spatially located tissues fed by each branch of the arterial vessel. For example, for each spatial location of an organ tissue represented by a voxel, values include a mapping to the arterial vessel branch and upstream branches that supply oxygenated blood.

A location of the stenosis 112 is identified at 310. The identification can include the generation of the CTA image and analysis using FFR-CT. The location of the stenosis 112 along the arterial vessel tree is identified, such as a branch along the coronary artery vessel tree. The location of the stenosis 112 can include a location of a stent or use other patient information to identify the location the stenosis 112.

At 320 the organ tissues fed downstream of the location of the stenosis 112 are determined from the feeding territory map 132. Axial positions of the determined organ tissues delineate the targeted coverage 200.

The targeted coverage 200 is input to the CT scanner 120 as parameters which control the coverage of the CT scanner 120 during a perfusion scan at 330. The perfusion scan scans the organ tissues within the targeted coverage 200 including during uptake of the contrast agent during the perfusion scan. A perfusion image is generated, which is a time dependent three dimensional image of the organ tissues scanned during the perfusion scan and can be displayed on the display device 160 and/or stored in the storage subsystem 150.

Modifications and alterations may occur to others upon reading and understanding the preceding detailed description.

## Claims

1. A system (100) for a targeted perfusion scan, comprising:
a computed tomography (CT) scanner (120) configured to perform a perfusion scan of a portion of tissues of an organ;
a feeding territory map (132) configured to map arterial locations of an arterial vessel tree to spatially located organ tissues of the organ fed by the arterial locations;
a stenosis unit (110) including one or more processors configured to determine the location of the stenosis from a computed tomography angiography (CTA) scan of the arterial vessel tree; and **characterised by**
a targeted perfusion unit (140) including the one or more processors (164) configured to determine targeted coverage (200) from a location of a stenosis (112, 210) and the feeding territory map, and to control the CT scanner to perform the perfusion scan according to the determined targeted coverage.

2. The system according to claim 1, further including:
a feeding territory map unit (130) including the one or more processors configured to generate the feeding territory map based on a sampling of one or more healthy organ arterial vessel trees and corresponding organ tissues.

3. The system according to any one of claims 1-2, wherein the targeted perfusion unit (140) is further configured to control the computed tomography (CT) scanner (120) to limit scanning to the targeted coverage during the perfusion scan.

4. The system according to any one of claims 1-3, wherein the targeted coverage includes positions in between a first axial position and a second axial position of the organ within a field of view of the CT scanner (120).

5. The system according to any one of claims 1-4, wherein the targeted coverage includes a distance between axial positions less than a field of view of the CT scanner (120).

6. The system according to any one of claims 1-5, wherein the feeding territory map (132) includes a mapping from each end branch of the arterial vessel tree hierarchically to connected upstream branches and mapping to the organ tissues feed by each branch of the arterial vessel tree.

7. The system according to any one of claims 1-6, wherein the feeding territory map (132) maps heart myocardial tissues fed by the coronary artery vessel tree.

8. The system according to any one of claims 2-7, wherein the feeding territory map unit (130) fits the feeding territory map (132) to the locations of the stenosis using an anatomical model (134) of the organ and arterial vessel tree.

9. A method of targeting a perfusion scan, comprising:
determining (320) targeted coverage (200) from a location of a stenosis (112, 210) and a feeding territory map (132) that maps arterial locations of an arterial vessel tree to spatially located organ tissues of the organ fed by the arterial locations;
determining (310) the location of the stenosis from a computed tomography angiography (CTA) scan of the arterial vessel tree; and
controlling (330) a computed tomography (CT) scanner (120) scan a portion of organ tissues according to the targeted coverage during the perfusion scan.

10. The method according to claim 9, further including:
generating (300) the feeding territory map (132) based on a sampling of one or more healthy organ arterial vessel trees and corresponding organ tissues.

11. The method according to any one of claims 9-10, wherein the targeted coverage includes positions in between a first axial position and a second axial position of the organ within a field of view of the CT scanner (120).

12. The method according to any one of claims 9-11, wherein the feeding territory map (132) includes a mapping from smallest branches of the arterial vessel tree hierarchically to larger branches and mapping to the organ tissues feed by each branch of the arterial vessel tree.

13. The method according to anyone of claims 9-12, wherein the feeding territory map(132) maps heart myocardial tissues fed by the coronary artery vessel tree.

14. The method according to any one of claims 9-13, wherein the feeding territory map unit fits the feeding territory map (132) to the locations of the stenosis using an anatomical model (134) of the organ and arterial vessel tree.

15. A non-transitory computer readable storage medium containing instructions that when executed by one or more processors:
determine (310) the location of the stenosis from a computed tomography angiography (CTA) scan of the arterial vessel tree;
determine a targeted coverage (200) of a computed tomography (CT) scanner from a location of a stenosis (112, 210) in an arterial vessel tree of an organ and a feeding territory map (132) that maps arterial locations of the arterial vessel tree to spatially located organ tissues of the organ fed by the arterial locations; and
control (330) the CT scanner to perform a targeted CT perfusion (CTP) scan according to the targeted coverage.

## Patentansprüche

1. Ein System (100) für einen gezielten Perfusions-Scan, das Folgendes umfasst:
einen Computertomographie (CT)-Scanner (120), der so konfiguriert ist, dass er einen Perfusions-Scan eines Teils von Geweben eines Organs durchführt; eine Versorgungsgebietskarte (132), die so konfiguriert ist, dass sie arterielle Orte eines arteriellen Gefäßbaums zu räumlich angeordneten Organgeweben des Organs, die von den arteriellen Stellen versorgt werden, abbildet; eine Stenose-Einheit (110), die einen oder mehrere Prozessoren enthält, die so konfiguriert sind, dass sie den Ort der Stenose aus einem Computertomographie-Angiographie (CTA)-Scan des arteriellen Gefäßbaums bestimmen; und **gekennzeichnet durch**
eine gezielte Perfusionseinheit (140), die den einen oder die mehreren Prozessoren (164) enthält, die so konfiguriert sind, dass sie die gezielte Abdeckung (200) aus dem Ort einer Stenose (112, 210) und der Karte des Versorgungsgebiets bestimmen und den CT-Scanner so steuern, dass er den Perfusions-Scan entsprechend der bestimmten gezielten Abdeckung durchführt.

2. System nach Anspruch 1, das ferner Folgendes umfasst: eine Versorgungsgebietskarteneinheit (130), die den einen oder die mehreren Prozessoren enthält, die so konfiguriert sind, dass sie die Versorgungsgebietskarte auf der Grundlage einer Probenahme von einem oder mehreren gesunden arteriellen Organ-Gefäßbäumen und entsprechenden Organgeweben erzeugen.

3. System nach einem der Ansprüche 1 bis 2, wobei die Einheit für gezielte Perfusioneinheit (140) des Weiteren so konfiguriert ist, dass sie den Computertomographie (CT)-Scanner (120) so steuert, dass das Scannen während des Perfusions-Scans auf die gezielte Abdeckung beschränkt wird.

4. System nach einem der Ansprüche 1 bis 3, wobei die gezielte Abdeckung Positionen zwischen einer ersten axialen Position und einer zweiten axialen Position des Organs innerhalb eines Sichtfeldes des CT-Scanners (120) umfasst.

5. System nach einem der Ansprüche 1-4, wobei die gezielte Abdeckung einen Abstand zwischen axialen Positionen einschließt, der kleiner ist als das Sichtfeld des CT-Scanners (120).

6. System nach einem der Ansprüche 1 bis 5, wobei die Karte des Versorgungsgebiets (132) eine Abbildung von jedem Endzweig des arteriellen Gefäßbaums hierarchisch zu den verbundenen stromaufwärts gelegenen Zweigen und eine Abbildung zu den Organgeweben, die von jedem Zweig des arteriellen Gefäßbaums versorgt werden, enthält.

7. System nach einem der Ansprüche 1 bis 6, wobei die Karte des Versorgungsgebiets (132) Herzmuskelgewebe abbildet, das durch den Koronararterien-Gefäßbaum versorgt wird.

8. System nach einem der Ansprüche 2 bis 7, wobei die Versorgungsgebietskarteneinheit (130) die Versorgungsgebietskarte (132) unter Verwendung eines anatomischen Modells (134) des Organs und des arteriellen Gefäßbaums an die Orte der Stenose anpasst.

9. Verfahren zur Ausrichtung eines Perfusions-Scans, das Folgendes umfasst: Bestimmen (320) einer Zielabdeckung (200) aus einem Ort einer Stenose (112, 210) und einer Versorgungsgebietskarte (132), die arterielle Orte eines arteriellen Gefäßbaums zu räumlich angeordneten Organgeweben des Organs, das von den arteriellen Orten versorgt wird, abbildet; Bestimmen (310) des Ortes der Stenose aus einem Computertomographie-Angiographie (CTA)-Scan des arteriellen Gefäßbaums; und Steuern (330) eines Computertomographie (CT)-Scanners (120), der einen Teil der Organgewebe gemäß der Zielabdeckung während des Perfusions-Scans scannt.

10. Verfahren nach Anspruch 9, das ferner Folgendes umfasst: Erzeugen (300) der Karte des Ernährungsgebiets (132) auf der Grundlage einer Stichprobe von einem oder mehreren gesunden arteriellen Organgefäßbäumen und entsprechenden Organgeweben.

11. Verfahren nach einem der Ansprüche 9-10, wobei die gezielte Abdeckung Positionen zwischen einer ersten axialen Position und einer zweiten axialen Position des Organs innerhalb des Sichtfeldes des CT-Scanners (120) umfasst.

12. Verfahren nach einem der Ansprüche 9 bis 11, wobei die Karte des Versorgungsgebiets (132) eine Zuordnung von kleinsten Ästen des arteriellen Gefäßbaums hierarchisch zu größeren Ästen und eine Zuordnung zu den Organgeweben, die von jedem Ast des arteriellen Gefäßbaums versorgt werden,
enthält.

13. Verfahren nach einem der Ansprüche 9 bis 12, wobei die Versorgungsgebietskarte (132) Herzmuskelgewebe abbildet, die von dem Koronararterien-Gefäßbaum versorgt werden.

14. Verfahren nach einem der Ansprüche 9 bis 13, wobei die Versorgungsgebietskarteneinheit
die Versorgungsgebietskarte (132) unter Verwendung eines anatomischen Modells (134) des Organs und des arteriellen Gefäßbaums an die Orte der Stenose anpasst.

15. Ein nicht-transitorisches, computerlesbares Speichermedium, das Anweisungen enthält, die bei Ausführung durch einen oder mehrere Prozessoren:
den Ort der Stenose aus einem Computertomographie-Angiographie (CTA)-Scan des arteriellen Gefäßbaums bestimmt (310); eine gezielte Abdeckung (200) eines Computertomographie (CT)-Scanners aus einem Ort einer Stenose (112, 210) in einem arteriellen Gefäßbaum eines Organs und einer Versorgungsgebietskarte (132) bestimmt, die die arteriellen Orte des arteriellen Gefäßbaums zu räumlich angeordneten
Organgeweben des Organs zuordnet, die von den arteriellen Orten versorgt werden; und Steuern (330) des CT-Scanners, um einen gezielten CT-Perfusions-Scan (CTP) entsprechend der gezielten Abdeckung durchzuführen.
according to the targeted coverage.

## Revendications

1. Système (100) pour un balayage de perfusion ciblé, comprenant:
un scanner (120) de tomographie par ordinateur (CT) configuré pour effectuer un balayage de perfusion d'une partie des tissus d'un organe;
une carte de territoire d'alimentation (132) configurée pour cartographier des emplacements artériels d'un arbre de vaisseaux artériels vers des tissus de l'organe situés dans l'espace et alimentés par les emplacements artériels;
une unité de sténose (110) comprenant un ou plusieurs processeurs configurés pour déterminer l'emplacement de la sténose à partir d'un balayage d'angiographie par tomographie informatisée (CTA) de l'arbre des vaisseaux artériels; et **caractérisée par** une unité de perfusion ciblée (140) comprenant un ou
plusieurs processeurs (164) configurés pour déterminer une couverture ciblée (200) à partir d'un emplacement d'une sténose (112, 210) et de la carte du territoire d'alimentation, et pour commander le CT scanner pour effectuer le balayage de perfusion selon la couverture ciblée déterminée.

2. Système selon la revendication 1, comprenant en outre:
une unité de carte de territoire d'alimentation (130) comprenant un ou plusieurs processeurs configurés pour générer la carte de territoire d'alimentation sur la base d'un échantillonnage d'un ou plusieurs arbres de vaisseaux artériels d'organes sains et de tissus d'organes correspondants.

3. Système selon l'une quelconque des revendications 1 à 2, dans lequel l'unité de perfusion ciblée (140) est en outre configurée pour commander le scanner de tomographie par ordinateur (CT) (120) afin de limiter le balayage à la couverture ciblée pendant le balayage de perfusion.

4. Système selon l'une quelconque des revendications 1 à 3, dans lequel la couverture ciblée comprend des positions entre une première position axiale et une deuxième position axiale de l'organe dans un champ de vision du CT scanner (120).

5. Système selon l'une quelconque des revendications 1 à 4, dans lequel la couverture ciblée comprend une distance entre des positions axiales inférieure à un champ de vision du CT scanner (120).

6. Système selon l'une quelconque des revendications 1 à 5, dans lequel la carte du territoire d'alimentation (132) comprend une cartographie de chaque branche terminale de l'arbre des vaisseaux artériels hiérarchiquement aux branches amont connectées et une cartographie des tissus organiques alimentés par chaque branche de l'arbre des vaisseaux artériels.

7. Système selon l'une quelconque des revendications 1 à 6, dans lequel la carte du territoire d'alimentation (132) cartographie les tissus myocardiques cardiaques alimentés par l'arbre des vaisseaux de l'artère coronaire.

8. Système selon l'une quelconque des revendications 2 à 7, dans lequel l'unité de carte de territoire d'alimentation (130) adapte la carte de territoire d'alimentation (132) aux emplacements de la sténose en utilisant un modèle anatomique (134) de l'organe et de l'arbre des vaisseaux artériels.

9. Procédé de ciblage d'un balayage de perfusion, comprenant:
déterminer (320) une couverture ciblée (200) à partir d'un emplacement d'une sténose (112, 210) et une carte de territoire d'alimentation (132) qui cartographie des emplacements artériels d'un arbre de vaisseaux artériels à des tissus d'organe situés dans l'espace de l'organe alimenté par les emplacements artériels;
déterminer (310) l'emplacement de la sténose à partir d'un balayage d'angiographie par tomographie informatisée (CTA) de l'arbre vasculaire artériel; et
commander (330) un scanner (120) de tomographie par ordinateur (CT) pour balayer une partie des tissus de l'organe selon la couverture ciblée pendant le balayage de perfusion.

10. Procédé selon la revendication 9, comprenant en outre:
la génération (300) de la carte de territoire d'alimentation (132) sur la base d'un échantillonnage d'un ou plusieurs arbres de vaisseaux artériels d'organes sains et de tissus d'organes correspondants.

11. Procédé selon l'une quelconque des revendications 9 à 10, dans lequel la couverture ciblée comprend des positions entre une première position axiale et une deuxième position axiale de l'organe dans un champ de vision du CT scanner (120).

12. Procédé selon l'une quelconque des revendications 9-11, dans lequel la carte du territoire d'alimentation (132) comprend une cartographie des plus petites branches de l'arbre des vaisseaux artériels hiérarchiquement vers les plus grandes branches et une cartographie des tissus organiques alimentés par chaque branche de l'arbre des vaisseaux artériels.

13. Procédé selon l'une quelconque des revendications 9 à 12, dans lequel la carte du territoire d'alimentation (132) cartographie les tissus du myocarde cardiaque alimentés par l'arbre vasculaire de l'artère coronaire.

14. Procédé selon l'une quelconque des revendications 9 à 13, dans lequel l'unité de carte de territoire d'alimentation adapte la carte de territoire d'alimentation (132) aux emplacements de la sténose en utilisant un modèle anatomique (134) de l'organe et de l'arbre vasculaire artériel.

15. Support de stockage lisible par ordinateur non transitoire contenant des instructions qui, lorsqu'elles sont exécutées par un ou plusieurs processeurs:
déterminent (310) l'emplacement de la sténose à partir d'un balayage d'angiographie par tomographie informatisée (CTA) de l'arbre vasculaire artériel;
déterminer une couverture ciblée (200) d'un scanner de tomographie par ordinateur (CT) à partir d'un emplacement d'une sténose (112, 210) dans un arbre vasculaire artériel d'un organe et d'une carte de territoire d'alimentation (132) qui cartographie les emplacements artériels de l'arbre vasculaire artériel vers des tissus d'organe situés dans l'espace de l'organe alimenté par les emplacements artériels; et
commander (330) le CT scanner pour effectuer un balayage de perfusion CT ciblé (CTP) selon la couverture ciblée.
